# EUROPEAN PATENT APPLICATION

(11) **EP 2 014 362 A1**
(43) Date of publication of application: **14.01.2009**
(21) Application number: 07110077.0
(22) Date of filing: 12.06.2007
(51) Int. Cl.: B01J 27/10, C07C 17/35, C07C 19/04

(54) **Methathesis of chlorinated waste compounds**

(71) Applicant: Universiteit Utrecht Holding B.V., 3584 CM Utrecht (NL)
(72) Inventor: van der Heijden, Aloysius Wilhemus Antonius Maria, 5226 EN Den Bosch (NL); Bitter, Johannes Hendrik, 3984 NN Odijk (NL); Weckhuysen, Bert Marc, 3994 BV Houten (NL)
(74) Representative: van Loon, C.J.J.

(57) **Abstract**

Metathesis process for a mixture of chlorinated hydrocarbons, which process comprises reacting a mixture of two or more different chlorinated hydrocarbons in the presence of at least one halogenide of a lanthanide, present on carbon as support.

## Description

In the last decades, chlorinated hydrocarbons (CHCs) have been replaced by environmentally friendlier alternatives in many commercial applications. However, mixtures of chlorinated C₁ and C₂ are still formed as by-products in industrial processes, such as the production of vinylchloride monomer (VCM) and the use thereof. CCl₄ formation is particularly a problem because of its lower economic value. Moreover, the United States Clean Air Act and the Montreal Protocol limit the production of CCl₄. Therefore, the metathesis reaction of CCl₄ with other CHCs, such as CH₂Cl₂, would be advantageous to make more valuable CHCs, which could be recycled in the production process.

Reaction thermodynamics are favorable for using CCl₄ as a Cl source with methane or chloromethane. The only known way for running these reactions is thermal gas phase radical chemistry. The disadvantage of radical chemistry is its low selectivity due to the formation of various chloromethanes and C₂ coupling products. In addition, coke formation at the temperatures that are required for radical generation is severely hampering the process. As a result, commercial application of this reaction is currently not feasible and incineration is commonly used as a route for the destruction of CCl₄.

This reaction is defined as a metathesis reaction by IUPAC, that is a bimolecular process formally involving the exchange of a bond (or bonds) between similar interacting chemical species so that the bonding affiliations in the products are identical (or closely similar) to those in the reactants.

It is an object to develop an improved process for the metathesis reaction of chlorinated hydrocarbons, such as those described above, or others, such as oligomers of VCM.

Research has been done to determine if this metathesis reaction can be improved by catalysis, especially with respect to selectivity, activity and required reaction conditions.

It has been found that this reaction can be catalyzed by the halogenides of the lanthanides, more in particular by lanthanum-chloride (LaCl₃). Although these catalyst materials improve the selectivity and activity, further improvement would be useful.

The use of carbon as support for the above mentioned catalyst has turned out to result in an important improvement in the reaction, more in particular in that the activity and selectivity improve. Further the harsh reaction conditions present fewer problems for this catalyst support.

Accordingly the invention is directed to a metathesis process for a mixture of chlorinated hydrocarbons, which process comprises reacting a mixture of two or more different chlorinated hydrocarbons in the presence of at least one halogenide of a lanthanide, present on carbon as support. More in particular a mixture of two or more different hydrocarbons is reacted in the presence of said catalyst, to produce one or more other chlorinated hydrocarbons.

As is shown in the examples and the attached figures, the present process provides a strongly improved activity for the metathesis reaction.

In an exemplary embodiment, the metathesis reaction according to the invention proceeds via net exchange of H and Cl atoms between CCl₄ and CH₂Cl₂ (Reaction 1). The catalytic reaction can be envisaged as two separate H/Cl exchange reactions between the catalyst surface and a gas phase reactant (Reaction 1 and 2).

CH₂Cl₂ (g) + Cl-LaCl₂ (s) → CHCl₃ (g) + H-LaCl₂ (s) (1)

CCl₄ (g) + H-LaCl₂ (s) → CHCl₃ (g) + Cl-LaCl₂ (s) (2)

The catalyst to be used in the process of the present invention has two essential components, the first one being the catalytically active material, which is in the present case a halogenide of a lanthanide. All halogenides may be used, but is is preferred to use a fluoride, chloride or bromide, with a preference for the chloride. Likewise all lanthanide metals may be used with the highest preference being given to lanthanum, preferably in the form of the trichloride.

The support material to be used in the present invention is carbon. All forms of carbon that can be used as catalyst support are suitable, the actual type being determined by the actual reaction conditions (gas-phase, slurry phase, fixed bed, fluidized bed, and the like), as well as the required loading with the catalytically active material and the required surface area.

Suitable forms of carbon are, without being limited, granular (activated) carbon, extrudated (activated) carbon, carbon fibres, carbon nano fibres (CNF) or monoliths. It is to be noted that instead of carbon it is also possible to use carbide (s).

The amount of catalytically active material may vary between wide ranges. Preferred amounts are between 1 and 35 wt.%, based on the total weight of the catalyst.

To achieve high conversion both on weight and volume basis, most preferably LaCl₃ is used, supported on CNF, thereby greatly increasing the surface area in comparison to bulk LaCl₃ catalysts. The reaction is suited for the efficient conversion of CCl₄, which is a highly stable and toxic compound with low economic value, as well as other highly chlorinated hydrocarbons.

The lanthanide-based catalysts are preferably synthesized by halogenation of a precursor of the lanthanide-halogenide salt to exclude the presence of lattice oxygen in the catalyst. This is required to prevent the destructive adsorption processes of the reactant molecules.

For a first set of experiments, a bulk LaCl₃ catalyst was synthesized using LaCl3 • 7H₂O, which was chlorinated using CCl₄. The catalyst was used for the metathesis between CCl₄ and CH₂Cl₂ (Reaction 3).

CH₂Cl₂ (g) + CCl₄ (g) → 2 CHCl₃ (g) (3)

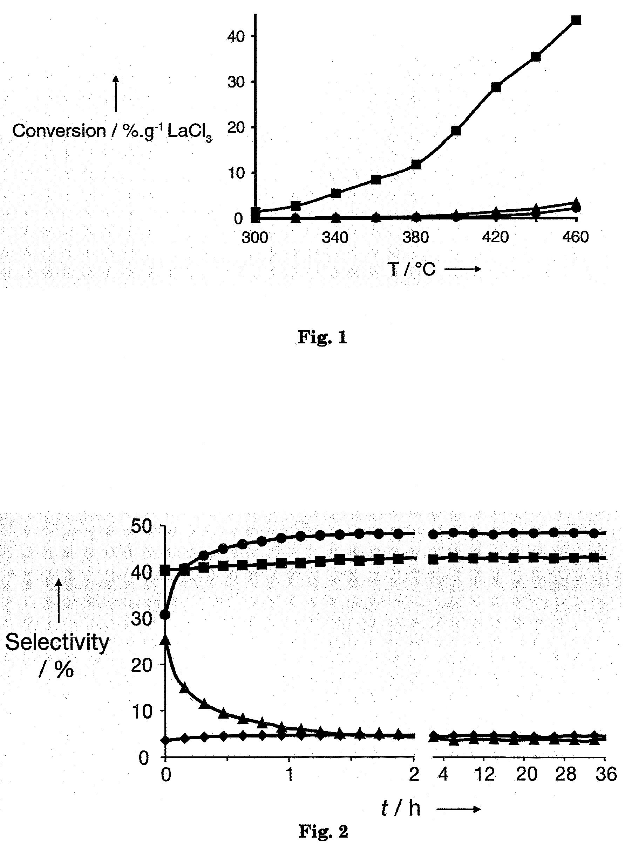

Figure 1 shows that this catalyst has a low, but appreciable activity with a 100 % selectivity towards CHCl₃ (not shown).

Although determination of the catalyst surface area is impracticable due to the hygroscopic nature of LaCl₃, the low conversion is most likely a result of the low surface area of the bulk catalyst. Therefore, LaOCl with a relatively high surface area (20-30 m²/g) was used as a precursor and was chlorinated with HCl. This resulted only in a slight increase in catalytic conversion, as shown in Figure 1. The higher surface area of the LaOCl precursor is most likely lost by the chlorination pre-treatment with HCl.

To efficiently increase the catalyst surface area, LaCl₃ was impregnated onto carbon nanofibers (CNF). This support was selected because of its relative inertness to the chlorination of the precursor with HCl and the absence of lattice oxygen, which may lead to destructive adsorption of the reactants.
Figure 1 clearly shows that this catalyst exhibits the highest conversion on a weight basis. Also on a volume basis, the CNF supported catalyst is at least two factors more active compared to the unsupported material (not shown).

To evaluate the stability of the novel LaCl₃/CNF catalyst material, the reaction was run for 36 h at 400 °C. The results are shown in Figure 2 and indicate the presence of an induction period in the first hour of reaction. In that period a significant amount of coke is formed together with HCl and Cl₂ (Reactions 4 and 5).

CCl₄ (g) → C (s) + 2 Cl₂ (g) (4)

CH₂Cl₂ (g) → C (s) + 2 HCl (g) (5)

Nevertheless, the selectivity to CHCl₃ remains constant. A possible explanation for the induction period is that Ni remaining from the CNF growth catalyzes the conversion of CCl₄ into coke. CNF are grown on a Ni/SiO₂ catalyst. After CNF have been grown Ni and SiO₂ are removed by washing steps. However, a trace amount of Ni is encapsulated in the CNF and can therefore not be removed by washing. During the metathesis experiments it may become accessible as a result of thermal expansion and the severe chlorination treatment needed for the synthesis of LaCl₃. In that case, the induction period is a result of the Ni catalyzed dissociation of the reactants. Especially CCl₄ is dissociating during this period as shown in Figure 2.

The reaction is preferably carried out at a temperature between 200 and 600°C, depending on the nature of the actual chlorinated hydrocarbons to be converted. Reaction time and pressure can easily be determined by the skilled person on the basis of the actual circumstances (reaction type, reactants and the like).

The present invention is further directed to the novel catalyst that is suitable for use in the above reaction and which catalyst comprises at least one halogenide of a lanthanide, present on carbon as support, more preferably LaCl₃ on carbon nano fibres.

### Description of Figures

Fig. 1 shows the conversion of a mixture of CH₂Cl₂ and CCl₄ into CHCl₃ as a function of temperature over bulk LaCl₃, synthesized by chlorination of LaCl₃ • 7H₂O with CCl₄ (●), bulk LaCl₃, synthesized by chlorination of LaOCl with HCl (▲) and 20 wt% LaCl₃ supported on CNF (■). (GHSV = 400 h⁻¹, inlet concentration CCl₄ = CH₂Cl₂ = 47000 ppm)
Fig. 2 shows the reaction mixture composition for the metathesis of CCl₄/CH₂Cl₂ at 400°C over 20 wt% LaCl₃ supported on CNF as a function of time. Only C-containing compounds are shown, namely CCl₄ (●), CH₂Cl₂ (■), CHCl₃ (◆) and C (▲). The selectivity for carbon was calculated based on the concentration of the gas phase products. (GHSV = 400 h⁻¹, inlet concentration CCl₄ = CH₂Cl₂ = 47000 ppm).

### EXAMPLES

Three LaCl₃-based catalyst materials were investigated; two bulk catalysts and one supported on carbon nanofibres (CNF). The precursors used for the bulk materials were synthesized LaOCl and commercial LaClg·7H₂O (1 m²/g. The carbon supported catalyst was synthesized by the incipient wetness impregnation method making use of an aqueous LaCl₃ solution on synthesized CNF. In this manner, 20 wt% LaCl₃ was loaded on the CNF material. CNF were grown as described by Toebes et al (M. L. Toebes, J. M. P. van Heeswijk, J. H. Bitter, A. J. van Dillen, K. P. de Jong, Carbon 2004, 42, 307-315).

Supported Ni/SiO₂ catalyst with a metal loading of 5 wt% was used to grow low density CNF (140 m²/g), prepared by homogeneous deposition precipitation. After impregnation the catalyst was dried at 120 °C overnight. Before chlorination, all catalysts were heated at 500 °C in He for 6 h. Next, the precursors were chlorinated; LaCI₃• 7H₂O was chlorinated with CCl₄ for 30 h at 460°C. LaOCl and the CNF-supported catalyst were chlorinated with HCl at 460 °C for 20 and 4 h, respectively.

Equimolar mixtures of either CH₂Cl₂ or CH₃Cl with CCl₄ of 4-6 vol% each in He were led over the catalyst with a flow rate of 5 ml/min. The reactor consisted of a quartz tube with a catalyst sieve fraction (325-500 µm) in between quartz wool. As the catalysts materials were synthesized by in situ synthesis, the amount of LaOCl and LaCl₃ • 7H₂O was pre-calculated for 1 g of LaCl₃. In the case of the carbon supported catalyst, the reactor was filled with approximately the same volume as in the case of the bulk catalyst. For the bulk materials, blank experiments were performed with quartz pellets. For the supported catalyst, CNF were used for a blank experiment. During the temperature programmed experiments, the reactor temperature was raised from 300 to 460 °C in steps of 20 °C, and the effluent was continuously analyzed by GC. In addition, the metathesis of CCl₄/CH₂Cl₂ was performed over the CNF supported catalyst at 400 °C as a function of time.

## Claims

1. Metathesis process for a mixture of chlorinated hydrocarbons, which process comprises reacting a mixture of two or more different chlorinated hydrocarbons in the presence of a catalyst based on at least one halogenide of a lanthanide, present on carbon as support.

2. Process according to claim 1, wherein a mixture of two or more different hydrocarbons is reacted in the presence of said catalyst, to produce one or more other chlorinated hydrocarbons.

3. Process according to claim 1 or 2, wherein the halogenide is selected form chloride, bromide and fluoride.

4. Process according to claim 1-3, wherein the lanthanide is lanthanum, preferably in the form of LaCl₃.

5. Process according to claim 1-4, wherein the carbon is in the form of granular (activated) carbon, extrudated (activated) carbon, carbon fibres, carbon nano fibres or a monolith.

6. Process according to claim 1-5, wherein the amount of the halogenide of a lanthanide, is between 1 and 35 wt. % based on the total weight of the catalyst.

7. Process according to claim 1-6, wherein the reaction is carried out at a temperature between 200 and 600°C.

8. Catalyst suitable for the metathesis reaction of a mixture of chlorinated hydrocarbons, comprising at least one halogenide of a lanthanide, present on carbon as support.

9. Catalyst according to claim 8, comprising LaCl₃ on carbon nano fibres.
